Europäisches Patentamt

European Patent Office

Office européen des brevets

(11) Veröffentlichungsnummer: **0 495 204 B1**

(12) **EUROPÄISCHE PATENTSCHRIFT**

(45) Veröffentlichungstag der Patentschrift: **14.06.95**

(51) Int. Cl.6: **C07D 475/04**, A61K 31/495

(21) Anmeldenummer: **91121326.2**

(22) Anmeldetag: **12.12.91**

Die Akte enthält technische Angaben, die nach dem Eingang der Anmeldung eingereicht wurden und die nicht in dieser Patentschrift enthalten sind.

(54) **Verfahren zur Herstellung von (6S)- und (6R)-Tetrahydrofolsäure.**

(30) Priorität: **16.01.91 CH 108/91**

(43) Veröffentlichungstag der Anmeldung:
**22.07.92 Patentblatt 92/30**

(45) Bekanntmachung des Hinweises auf die Patenterteilung:
**14.06.95 Patentblatt 95/24**

(84) Benannte Vertragsstaaten:
**AT BE CH DE DK ES FR GB GR IT LI LU NL SE**

(56) Entgegenhaltungen:
**EP-A- 0 266 042**
**EP-A- 0 293 029**
**EP-A- 0 348 641**
**EP-A- 0 356 934**
**EP-A- 0 367 902**

**JOURNAL OF MEDICINAL CHEMISTRY. Bd. 22, Nr. 6, Juni 1979, WASHINGTON US Seiten 731 - 734; C. TEMPLE, JR., ET AL.: 'Preparation and Purification of ...'**

**TETRAHEDRON, Bd. 42, Nr. 1, 1986, OXFORD GB Seiten 117 - 136; L. REES ET AL.: 'Asymmetric reduction of dihydrofolate ...'**

**HELV.CHIM ACTE, 1974, S 2658-61**

(73) Patentinhaber: **EPROVA Aktiengesellschaft**
**Im Laternenacker 5**
**CH-8200 Schaffhausen (CH)**

(72) Erfinder: **Müller, Hans Rudolf**
**Beckenwäldli 18**
**CH-8207 Schaffhausen (CH)**
Erfinder: **Ulmann, Martin**
**Steigstrasse 36**
**CH-8447 Dachsen (CH)**
Erfinder: **Conti, Josef**
**Winkelriedstrasse 22**
**CH-8203 Schaffhausen (CH)**
Erfinder: **Mürdel, Günter**
**vor Hegin**
**W-7708 Tengen-Büsslingen (DE)**

**Beschreibung**

Die Erfindung betrifft ein Verfahren zur Herstellung von N-[4-[[(2-amino-1,4,5,6,7,8-hexahydro-4-oxo-(6S)-pteridinyl)methyl]amino]benzoyl]-L-glutaminsäure (im folgenden (6S)-Tetrahydrofolsäure genannt) und deren Salzen und N-[4-[[(2-amino-1,4,5,6,7,8-hexahydro-4-oxo-(6R)-pteridinyl)methyl]amino]benzoyl]-L-glutaminsäure (im folgenden (6R)-Tetrahydrofolsäure genannt) und deren Salzen.

Tetrahydrofolsäurederivate enthalten 2 asymmetrische Zentren. Dabei liegt aufgrund der Synthese dieser Derivate aus Folsäure, der N-(Pteroyl)-L-glutaminsäure, das im Glutaminsäure-Rest enthaltene optisch aktive C-Atom in der L-Form vor, während das durch Hydrierung der Doppelbindung in 5,6-Stellung des Pteroyl-Restes entstandene optisch aktive C-Atom in Position 6 in der racemischen, der (6R,S)-Form, vorliegt. Synthetische Derivate der Tetrahydrofolsäure bestehen demnach aus einer 1:1 Mischung von 2 Diastereomeren. Im natürlichen Vorkommen, z.B. in der Leber, findet man die Tetrahydrofolate nur in der einen Diastereomeren-Form, wobei 5,6,7,8-Tetrahydrofolsäure in der (6S)-Form vorliegt.

Als Arzneimittel werden Tetrahydrofolate vorwiegend als Calcium-Salz der 5-Formyl-5,6,7,8-tetrahydrofolsäure (Leucovorin) oder der 5-Methyl-5,6,7,8-tetrahydrofolsäure verwendet zur Behandlung von megaloblastischer Folsäure-Anämie, als Antidot zur Verstärkung der Verträglichkeit von Folsäure-Antagonisten, speziell von Aminopterin und Methotrexat in der Krebstherapie ("leucovorin rescue"), zur Verstärkung des therapeutischen Effektes von 5-Fluoruracil und der Behandlung von Autoimmunkrankheiten wie Psoriasis und rheumatischer Arthritis sowie zur Verstärkung der Verträglichkeit von bestimmten Antiparasitika, etwa Trimethoprim-Sulfamethoxazol, in der Chemotherapie. Tetrahydrofolsäure dient als Grundsubstanz zur Herstellung diverser Tetrahydrofolsäure-Derivate.

Anstrengungen zur Herstellung von (6S)-oder (6R)-Tetrahydrofolsäure haben bisher zu folgenden Methoden geführt:
- enzymatische Methoden
- physikochemische Methoden
- chemische Methoden

Enzymatische Methoden beinhalten eine normalerweise chemisch durchgeführte Reduktion von Folsäure zu 7,8-Dihydrofolsäure und anschliessender enzymatischer Reduktion derselben zu (6S)-5,6,7,8-Tetrahydrofolsäure z.B. gemäss L. Rees et al, Tetrahedron **42**(1), 117-36 (1986) oder EP-A2-0 356 934. Diese Verfahren sind jedoch im chemischen Schritt nur schwer auf der 7,8-Dihydrofolsäure-Stufe zu stoppen und ergeben auch im enzymatischen Schritt typischerweise nur sehr geringe Raum-Zeit-Ausbeuten, benötigen teure Co-Faktoren wie NADPH und erfordern eine meist komplexe Aufarbeitungsmethodik. Bisher bekannte Methoden der enzymatischen Herstellung von optisch reiner Tetrahydrofolsäure sind zur Herstellung dieser Verbindung im technischen Massstab nicht geeignet.

Die Trennung der Diastereomeren-Paare wurde auch mittels Chromatographie versucht, J. Feeney et al, Biochemistry, **20**, 1837, (1981). Diese Methoden sind für die Herstellung der Diastereomeren im technischen Massstab nicht geeignet.

Ebenfalls bekannt ist aus der Gruppe der physikochemischen Verfahren eine asymmetrische Reduktion von Folsäure an chiralen Elektroden, S. Kwee et al, Bioelectrochem. Bioenerg. **7**, 693-698, (1980). Aufgrund der während der Reduktion erlaubten Konzentrationen an Folsäure (typischerweise $10^{-3}$ M) und der nur schwer durchführbaren Abtrennung des asymmetrischen Induktors nach erfolgter Reduktion können diese Reaktionen jedoch nicht zur technischen Herstellung eingesetzt werden.

Aus dem Bereich der chemischen Synthese besteht die Möglichkeit der asymmetrischen Hydrierung von Folsäure in Gegenwart eines optisch aktiven Katalysators, etwa gemäss P.H. Boyle, et al., J. Chem. Soc. Chem. Commun. (1974), **10**, 375-6. Diese erfordert aber den Einsatz sehr teurer Katalysatoren, die nach erfolgter homogener Katalyse nur unter grossem Aufwand vom Produkt abzutrennen sind.

Im Bereich der Herstellung und Trennung von Tetrahydrofolsäure-Derivaten sind verschiedene Verfahren bekannt. So beschreibt EP-A-0 293 029 ein Verfahren zur Trennung von 5-Formyl-(6R,S)-tetrahydrofolsäure bei erhöhtem pH-Wert. EP-A-0 367 902 offenbart die Trennung von 5-Formyl-(6R,S)-tetrahydrofolsäure durch Zugabe von Alkali-, Ammonium- oder Erdalkalisalzen. EP-A-0 348 641 betrifft ein Verfahren zur Trennung der sauren Salze oder des inneren Salzes der 5,10-Methenyl-(6R,S)-tetrahydrofolsäure. In EP-A-0 266 042 wird ein Trennverfahren für 5-(-)-Menthyloxycarbonyl-, 5-(-)-Bornyloxycarbonyl- und 5-(-)-Isobornyloxycarbonyl-(6R,S)-tetrahydrofolsäure beschrieben. Alle diese Methoden erfordern jedoch weitere chemische Verfahrensschritte zur Umsetzung der erhaltenen Tetrahydrofolsäure-Derivate zu optisch reiner Tetrahydrofolsäure.

Es gibt daher bis heute noch kein technisch brauchbares Verfahren zur Gewinnung von optisch reiner Tetrahydrofolsäure.

Es bestand somit die Aufgabe, eine einfache, technisch brauchbare und wirtschaftliche Methode zur Herstellung von optisch reiner Tetrahydrofolsäure zu erarbeiten.

Es wurde nun überraschend gefunden, dass aus wässrigen Lösungen von (6R,S)-Tetrahydrofolsäure oder deren Salze nach Zusatz von Sulfonsäuren oder Schwefelsäure sich entsprechendes optisch angereichertes Tetrahydrofolsäure-Additionssalz ausscheidet. Dieses lässt sich durch Filtration abtrennen. Aus dem Filtrat lässt sich das diastereomere Additionssalz isolieren. Beide Salze können anschliessend durch Umkristallisation und/oder Freisetzung der Tetrahydrofolsäure und nachfolgender Versalzung sowohl chemisch wie auch optisch gereinigt werden. Dass bei der Kristallisation des Sulfonsäure- bzw. Schwefelsäure-Additionssalzes eine optische Auftrennung stattfindet, ist um so mehr überraschender, als dass sich durch Herstellung/Umkristallisation anderer Salze, z.B. des Chlorwasserstoffsäure-Additionssalzes, keine optische Anreicherung feststellen lässt (W. Frick, et al., Helv. Chim. Acta, 57, 2658-61 (1974)). Auch mit andern starken Säuren wie Bromwasserstoffsäure, Iodwasserstoffsäure, Salpetersäure, Phosphorsäure, Ameisensäure, Oxalsäure, Chlor-, Dichlor- und Trichloressigsäure liess sich keine Anreicherung einer der epimeren Formen der Tetrahydrofolsäure erzielen.

Gegenstand der Erfindung ist ein Verfahren zur Herstellung von (6S)- und (6R)-Tetrahydrofolsäure sowie von deren Additionssalzen mit Sulfonsäuren oder mit Schwefelsäure, dadurch gekennzeichnet, dass man (6R,S)-Tetrahydrofolsäure mit einer Sulfonsäure oder mit Schwefelsäure umsetzt, erhaltenes Säureadditionssalz fraktioniert kristallisiert und, falls erwünscht, aus den erhaltenen diastereomeren Säureadditionssalzen durch Behandlung mit einer Base die (6S)- und/oder (6R)-Tetrahydrofolsäure freisetzt und isoliert.

Die dabei verwendete (6R,S)-Tetrahydrofolsäure kann sowohl als isoliertes Produkt oder aber auch vorzugsweise als Produkt einer Reduktion von Folsäure direkt in situ eingesetzt werden.

Für das erfindungsgemässe Verfahren geeignete Sulfonsäuren sind aromatische Sulfonsäuren mit 6 bis 14 C-Atomen, araliphatische Sulfonsäuren mit 7 bis 9 C-Atomen oder aliphatische Sulfonsäuren mit 1 bis 3 C-Atomen. Als aromatische Sulfonsäuren kommen vorwiegend in Betracht Benzolsulfonsäure, Toluolsulfonsäuren, Xylolsulfonsäuren, Nitrobenzolsulfonsäuren, Chlorbenzolsulfonsäuren, Nitrotoluolsulfonsäuren, Naphthalinsulfonsäuren, substituierte Naphthalinsulfonsäuren, Naphthalindisulfonsäuren, Camphersulfonsäuren, Benzimidazolsulfonsäuren, substituierte Benzimidazolsulfonsäuren, wie z. B. 2-Phenylbenzimidazol-5-sulfonsäure und viele andere mehr.

Als araliphatische Sulfonsäure kommt vorwiegend Phenylmethansulfonsäure, als aliphatische Sulfonsäuren vorwiegend Methansulfonsäure und Ethansulfonsäure in Betracht.

Bevorzugte Additionssalze für das erfindungsgemässe Verfahren sind das Benzolsulfonsäure-, ein Toluolsulfonsäure- sowie das Schwefelsäure-Additionssalz.

Die Kristallisation wird aus einem polaren Medium durchgeführt. Dabei eignet sich vor allem Wasser oder ein Gemisch aus Wasser und niedrigen aliphatischen wasserlöslichen Carbonsäuren, insbesondere Essigsäure sowie Milchsäure oder flüssigen wasserlöslichen Amiden wie Formamid, Dimethylformamid, Dimethylacetamid, 1-Methylpyrrolidon, 2-Piperidinon. Das Gemisch enthält üblicherweise mindestens 50% Wasser. Der Einsatz einer solchen Mischung erhöht normalerweise die optische Reinheit der Produkte, wobei jedoch die Ausbeute sinken kann. Abhängig vom angestrebten Ziel und dem jeweiligen Ausgangsmaterial kann das Optimum der Reaktionsbedingungen durch systematische Versuche ohne Schwierigkeiten bestimmt werden.

Aufgrund der Oxidationsempfindlichkeit der Tetrahydrofolsäure empfiehlt sich die Verwendung eines Oxidationsschutzmittels wie 2-Mercaptoethanol.

Bei der Kristallisation fällt in der Regel zuerst das Säureadditionssalz der (6S)-Tetrahydrofolsäure aus - die diastereomere (6R)-Verbindung reichert sich in den Filtraten an.

Aus den erhaltenen Salzen kann die optisch angereicherte Tetrahydrofolsäure sehr einfach durch Zusatz einer Base wieder freigesetzt werden.

Als weitere chemische wie auch optische Reinigungsmöglichkeit bietet sich die Umkristallisation der (6S)- bzw. (6R)-Tetrahydrofolsäure-Säureadditionssalze und/oder die anschliessend an die Freisetzung der Tetrahydrofolsäure erneut durchgeführte Versalzung mit einer Sulfonsäure und/oder Schwefelsäure an.

Durch dieses Verfahren sind die (6S)- und (6R)-Tetrahydrofolsäure und deren Salze mit starken Basen oder Säuren sehr einfach und besonders wirtschaftlich zugänglich geworden.

Die Erfindung betrifft auch die Verwendung der verfahrensgemäss erhaltenen (6S)-und (6R)-Tetrahydrofolsäure und deren Additionssalze mit Sulfonsäuren oder mit Schwefelsäuren zur Herstellung von optisch reinen 5,10-Methylentetrahydrofolsäuren und deren Salze mit starken Basen oder Säuren durch Behandlung mit Formaldehyd. Dabei ist zu beachten, dass aus (6S)-Tetrahydrofolsäure durch Umsetzung mit Formaldehyd 5,10-Methylen-(6R)-tetrahydrofolsäure und aus (6R)-Tetrahydrofolsäure analog 5,10-Methylen-(6S)-tetrahydrofolsäure erhalten wird.

**Beispiele zur Illustrierung der Erfindung:**

Zur Gehaltsbestimmung der Tetrahydrofolsäure, 5-Formyl-, 5-Methyl- und 5,10-Methylentetrahydrofolsäure wurde die folgende HPLC-Methode eingesetzt:

Fliessmittel A:

0,03 M $Na_2HPO_4$ + 0,03 M $KH_2PO_4$ in Wasser

Fliessmittel B:

1 Teil (0,03 M $Na_2HPO_4$ + 0,03 M $KH_2PO_4$ in Wasser)

3 Teile Methanol

danach mit Phosphorsäure auf pH 7,8 gestellt

Gradient:

innerhalb von 25 Minuten von 2% Fliessmittel B auf 95% Fliessmittel B

Säule:

ODS (Hypersil)

Detektion:

UV-300 nm

Zur Bestimmung des (6S)-Anteils der Tetrahydrofolsäure wurde die folgende HPLC-Methode eingesetzt:

Derivatisierung:

Tetrahydrofolsäure bzw. Additionssalz in Acetonitril/Wasser 1:1 lösen und mit 2,3,4,6-Tetra-o-acetyl-$\beta$-D-glucopyranosylisothiocyanat umsetzen

Fliessmittel:

2,5 Teile Acetonitril

1,5 Teile Methanol

6,0 Teile Citronensäure 0,02 M

Säule:

RP-8 (Lichrospher)

Detektion:

UV-270 nm

**Beispiel 1**

14,3 g Toluol-4-sulfonsäure (150 Mol%) werden bei 60° C unter Stickstoff in 440 ml Wasser, enthaltend 0,1% 2-Mercaptoethanol, gelöst. Innerhalb von 5 Minuten werden 25,0 g reine (6R,S)-Tetrahydrofolsäure eingetragen. Die entstandene Suspension wird auf 40° C abgekühlt. Nach 2 - 5 Stunden wird das ausgeschiedene Produkt abfiltriert, mit Wasser und danach mit Ethanol gewaschen.

Man erhält 16,9 g Toluol-4-sulfonsäur-Additionssalz der (6S)-Tetrahydrofolsäure mit einem (6S)-Anteil von 86,7%; bestimmt mittels HPLC.

Durch Umkristallisation des erhaltenen Produktes aus einer Mischung von 110 ml N,N'-Dimethylformamid und 220 ml Wasser erhält man Toluol-4-sulfonsäure-Additionssalz der (6S)-Tetrahydrofolsäure mit einem (6S)-Anteil von 97,5%; bestimmt mittels HPLC.

$[\alpha]_D^{25}$ = - 60,6° (c = 0,5% in DMF)

Zur Freisetzung der (6S)-Tetrahydrofolsäure werden 170 ml Wasser, enthaltend 0,1% 2-Mercaptoethanol, unter Stickstoff auf 10° C abgekühlt. 5,0 g Toluol-4-sulfonsäur-Additionssalz der (6S)-Tetrahydrofolsäure werden eingestreut. Zu dieser Suspension werden 4 ml 2n Natronlauge zugetropft.

Nach Abkühlen auf 2° C wird das ausgeschiedene Produkt abfiltriert, mit Wasser und danach mit Ethanol gewaschen.

Man erhält 3,7 g (6S)-Tetrahydrofolsäure mit einem (6S)-Anteil von 97,7%; bestimmt mittels HPLC.

$[\alpha]_D^{25}$ = - 44,5° (c = 1% in Wasser)

**Beispiel 2**

13 g Toluol-4-sulfonsäure (135 Mol%) werden bei 20° C unter Stickstoff in 200 ml Essigsäure und 200 ml Wasser, enthaltend 0,2% 2-Mercaptoethanol, gelöst. 25,0 g reine (6R,S)-Tetrahydrofolsäure werden rasch eingetragen. Die Lösung wird mit wenig authentischem (6S)-Tetrahydrofolsäuretoluol-4-sulfonsäur-Additionssalz angeimpft. Nach 5 Stunden wird das ausgeschiedene Produkt abfiltriert, mit Essigsäure/Wasser und danach mit Ethanol gewaschen.

Man erhält 12,7 g Toluol-4-sulfonsäur-Additionssalz der (6S)-Tetrahydrofolsäure mit einem (6S)-Anteil von 93,6%; bestimmt mittels HPLC.

10,0 g des so erhaltenen (6S)-Tetrahydrofolsäuretoluol-4-sulfonsäur-Additionssalzes werden bei 25° C unter Stickstoff in 100 ml Wasser suspendiert und mit Natronlauge 30% auf pH > 3,5 gestellt. Der pH der so erhaltenen Lösung wird danach wieder mit Salzsäure 37% auf unter 1 gebracht. Nach 12 Stunden wird das ausgeschiedene Produkt abfiltriert, mit Wasser und danach mit Ethanol gewaschen.

Man erhält 8,9 g Toluol-4-sulfonsäur-Additionssalz der (6S)-Tetrahydrofolsäure mit einem (6S)-Anteil von 99,7%; bestimmt mittels HPLC.

$[\alpha]_D^{25}$ = - 62,0° (c = 0,5% in DMF)

**Beispiel 3**

14,30 g Toluol-4-sulfonsäure (150 Mol%) werden bei 27° C unter Stickstoff in 220 ml L(+)-Milchsäure und 220 ml Wasser, enthaltend 0,2% Mercaptoethanol, gelöst. 25,0 g reine (6R,S)-Tetrahydrofolsäure werden rasch zugegeben. Die entstandene Lösung wird mit wenig authentischem (6S)-Tetrahydrofolsäure-toluol-4-sulfonsäur-Additionssalz angeimpft und auf 20° C gekühlt. Nach 15 - 20 Stunden wird das ausgeschiedene Produkt abfiltriert, mit Milchsäure/Wasser und danach mit Ethanol gewaschen.

Man erhält 15,1 g Toluol-4-sulfönsäur-Additionssalz der (6S)-Tetrahydrofolsäure mit einem (6S)-Anteil von 92,5%; bestimmt mittels HPLC.

**Beispiel 4**

14,3 g Toluol-4-sulfönsäure (150 Mol%) werden bei 27° C unter Stickstoff in 110 ml 1-Methyl-2-pyrrolidon und 110 ml Wasser, enthaltend 0,4% 2-Mercaptoethanol, gelöst. 25,0 g reine (6R,S)-Tetrahydrofolsäure werden rasch zugegeben. Die Lösung wird mit 220 ml Wasser verdünnt und auf 20°C abgekühlt. Nach 15 - 20 Stunden wird das ausgeschiedene Produkt abfiltriert, mit 1-Methyl-2-pyrrolidon/Wasser und danach mit Ethanol gewaschen.

Man erhält 13,3 g Toluol-4-sulfonsäur-Additionssalz der (6S)-Tetrahydrofolsäure mit einem (6S)-Anteil von 94,7%; bestimmt mittels HPLC.

**Beispiel 5**

11,5 g Toluol-4-sulfonsäure (150 Mol%) werden bei 27°C unter Stickstoff in 90 ml N,N'-Dimethylformamid und 90 ml Wasser, enthaltend 0,4% 2-Mercaptoethanol, gelöst. 20 g reine (6R,S)-Tetrahydrofolsäure werden rasch zugegeben. Die Lösung wird mit 180 ml Wasser verdünnt und auf 20°C abgekühlt. Nach 15 - 20 Stunden wird das ausgeschiedene Produkt abfiltriert, mit N,N'-Dimethylformamid/Wasser und danach mit Ethanol gewaschen.

Man erhält 11,3 g Toluol-4-sulfonsäure-Additionssalz der (6S)-Tetrahydrofolsäure mit einem (6S)-Anteil von 91,4%; bestimmt mittels HPLC.

**Beispiel 6**

11,5 g Toluol-4-sulfonsäure (150 Mol%) werden bei 27°C unter Stickstoff in 100 ml N,N'-Dimethylacetamid und 80 ml Wasser, enthaltend 0,4% 2-Mercaptoethanol, gelöst. 20 g reine (6R,S)-Tetrahydrofolsäure werden rasch zugegeben. Die Lösung wird mit 160 ml Wasser verdünnt und auf 20°C abgekühlt. Nach 15-20 Stunden wird das ausgeschiedene Produkt abfiltriert, mit N,N'-Dimethylacetamid/Wasser und danach mit Ethanol gewaschen.

Man erhält 11,0 g Toluol-4-sulfonsäur-Additionssalz der (6S)-Tetrahydrofolsäure mit einem (6S)-Anteil von 91,5%; bestimmt mittels HPLC.

**Beispiel 7**

12 g Benzolsulfonsäure (150 Mol%) werden bei 70°C unter Stickstoff in 440 ml Wasser, enthaltend 0,1% 2-Mercaptoethanol, gelöst. Innerhalb von 5 Minuten werden 25,0 g reine (6R,S)-Tetrahydrofolsäure eingetragen. Die entstandene Suspension wird auf 60°C abgekühlt. Nach 2 - 5 Stunden wird das ausgeschiedene Produkt abfiltriert, mit Wasser und danach mit Ethanol gewaschen.

Man erhält 13,8 g Benzolsulfonsäur-Additionssalz der (6S)-Tetrahydrofolsäure mit einem (6S)-Anteil von 92,4%; bestimmt mittels HPLC.

10,0 g des so erhaltenen (6S)-Tetrahydrofolsäurebenzolsulfonsäur-Additionssalzes werden bei 25°C unter Stickstoff in 100 ml Wasser suspendiert und mit Natronlauge 30% auf pH > 3,5 gestellt. Der pH der so erhaltenen Lösung wird danach wieder mit Salzsäure 37% auf unter 1 gebracht. Nach 12 Stunden wird das ausgeschiedene Produkt abfiltriert, mit Wasser und danach mit Ethanol gewaschen.

Man erhält 9,0 g Benzolsulfonsäure-Additionssalz der (6S)-Tetrahydrofolsäure mit einem (6S)-Anteil von 99,8%; bestimmt mittels HPLC.

$[\alpha]_D^{25} = -63,5°$ (c = 1% in DMF)

Bei dem Ersatz der eingesetzten 150 Mol% Benzolsulfonsäure durch 55 Mol% Benzolsulfonsäure und 50 Mol% Salzsäure erhält man unter gleichen Kristallisationsbedingungen 12,7 g Benzolsulfonsäur-Additionssalz der (6S)-Tetrahydrofolsäure mit einem (6S)-Anteil von 91,6%; bestimmt mittels HPLC.

Zur Freisetzung der (6R)-Tetrahydrofolsäure wird das Filtrat mit Natronlauge auf pH 3,5 gestellt. Nach Abkühlen auf 5°C wird das ausgeschiedene Produkt abfiltriert, mit Wasser und danach mit Ethanol gewaschen.

10 g des so erhaltenen Rückstandes werden in 150 ml Wasser, enthaltend 0,1% 2-Mercaptoethanol, und 39 ml 2N Schwefelsäure bei 50°C unter Stickstoff gelöst. Nach langsamem Abkühlen über 15 Stunden auf 20°C und anschliessendem 12-stündigem Stehenlassen wird das, ausgeschiedene Produkt abfiltriert, mit Wasser und danach mit Ethanol gewaschen.

Man erhält 9,7 g Schwefelsäur-Additionssalz der (6R)-Tetrahydrofolsäure mit einem (6R)-Anteil von 97,7%; bestimmt mittels HPLC.

**Beispiel 8**

30 ml 2 M Schwefelsäure werden bei 60°C mit 130 ml Wasser, enthaltend 0,2% 2-Mercaptoethanol, und 164 ml Eisessig vorgelegt. Innerhalb von 5 Minuten werden 20 g reine (6R,S)-Tetrahydrofolsäure eingetragen. Die entstandene Lösung wird auf 50°C abgekühlt. Nach 1 Stunde wird das ausgeschiedene Produkt abfiltriert, mit Wasser/Eisessig und danach mit Ethanol gewaschen.

Man erhält 11,0 g Schwefelsäur-Additionssalz der (6S)-Tetrahydrofolsäure mit einem (6S)-Anteil von 65,5%; bestimmt mittels HPLC.

Durch zweifaches Umkristallisieren von 10 g Schwefelsäure-Additionssalz der (6S)-Tetrahydrofolsäure aus Dimethylformamid/Wasser 1:3 erhält man 3,9 g Schwefelsäure-Additionssalz der (6S)-Tetrahydrofolsäure mit einem (6S)-Anteil von 94,3%; bestimmt mittels HPLC.

**Beispiel 9**

Nach in der Literatur beschriebenen Verfahren, z.B. R.L. Blakley et al, Folates and Pterins, **1**, 93-104 (1984) in situ hergestellte (6R,S)-Tetrahydrofolsäure wird direkt weiter mit Toluol-4-sulfonsäure umgesetzt:

50 g Folsäure werden etwa nach C. Temple, J. Med. Chem., **22**, 731 (1979) bei 25° C unter Stickstoff in 200 ml Wasser suspendiert. Mit ca. 40 g Natronlauge 30% wird der pH der Lösung auf 12 gestellt. Nach Zugabe von 25 g Natriumborhydrid (630 Mol%) in 110 ml Wasser wird das Reaktionsgemisch auf 70-75° C aufgeheizt und während 90 Minuten bei dieser Temperatur gehalten. In die so erhaltene Lösung von (6R,S)-Tetrahydrofolsäure-Natriumsalz werden nach Abkühlen auf 25° C 30 g Toluol-4-sulfonsäure (150 Mol%), gelöst in 200 ml Eisessig, zugetropft. Mit 96 g Salzsäure 37 %, wird danach der pH der Lösung auf unter 1 gestellt. Nach 12 Stunden wird das ausgeschiedene Produkt abfiltriert, mit Essigsäure/Wasser und danach mit Ethanol gewaschen.

Man erhält 32,9 g Toluol-4-sulfönsäur-Additionssalz der (6S)-Tetrahydrofolsäure mit einem Gehalt von 82% und einem (6S)-Anteil von 95,4%; bestimmt mittels HPLC.

3,0 g des so erhaltenen (6S)-Tetrahydrofolsäuretoluol-4-sulfonsäur-Additionssalzes werden bei 25° C unter Stickstoff in 30 ml Wasser suspendiert und mit ca. 3 g Natronlauge 30% auf pH 11,6 gestellt. Zur so erhaltenen Lösung werden 0,9 g Toluol-4-sulfonsäure (120 Mol%), gelöst in 36 ml Eisessig, zugetropft. Mit 2,2 g Salzsäure 37% wird danach der pH der Lösung auf unter 1 gestellt. Nach 12 Stunden wird das ausgeschiedene Produkt abfiltriert, mit Essigsäure/Wasser und danach mit Ethanol gewaschen.

Man erhält 1,81 g Toluol-4-sulfönsäur-Additionssalz der (6S)-Tetrahydrofolsäure mit einem Gehalt von 100% und einem 6S-Anteil von 98,9%; bestimmt mittels HPLC.

**Beispiel 10**

Durch den Ersatz der Toluol-4-sulfönsäure in Beispiel 9 durch die aequivalente Menge an Benzolsulfonsäure lässt sich in ähnlicher Weise auch das Benzolsulfonsäure-Additionssalz der (6S)-Tetrahydrofolsäure herstellen.

Man erhält 32,2 g Benzolsulfonsäure-Additionssalz der (6S)-Tetrahydrofolsäure mit einem Gehalt von 80% und einem (6S)-Anteil von 94,2%; bestimmt mittels HPLC.

**Beispiel 11**

Durch den Ersatz der Toluol-4-sulfonsäure in Beispiel 9 durch die aequivalente Menge an Schwefelsäure lässt sich in ähnlicher Weise auch das Sulfat der (6S)-Tetrahydrofolsäure herstellen.

Man erhält 27,1 g (6S)-Tetrahydrofolsäuresulfat mit einem Gehalt von 85% und einem (6S)-Anteil von 69,2%; bestimmt mittels HPLC.

**Beispiele 12 - 18**

In ähnlicher Weise, wie in den Beispielen 1 - 8 beschrieben, lassen sich herstellen:

12. Methansulfonsäure-Additionssalz der (6S)-Tetrahydrofolsäure.

13. Ethansulfonsäure-Additionssalz der (6S)-Tetrahydrofolsäure.

14. Phenylmethansulfonsäure-Additionssalz der (6S)-Tetrahydrofolsäure.

15. Campher-10-sulfonsäur-Additionssalz der (6S)-Tetrahydrofolsäure.

16. Naphthalin-1-sulfonsäure-Additionssalz der (6S)-Tetrahydrofolsäure.

17. Naphthalin-2-sulfonsäure-Additionssalz der (6S)-Tetrahydrofolsäure.

18. Naphthalin-1,5-disulfönsäur-Additionssalz der (6S)-Tetrahydrofolsäure.

**Beispiel 19**

50 g von nach Beispiel 8 erhaltenem Schwefelsäure-Additionssalz der (6S)-Tetrahydrofolsäure werden bei 20° C unter Stickstoff in 500 ml Wasser mit 200 ml Natronlauge 2N gelöst. Nach der Zugabe von 7,5 ml Formaldehyd 36% (125 Mol%) wird die Lösung mit einer Mischung von 275 ml Eisessig und 275 ml Schwefelsäure 2 N versetzt. Nach dem Kühlen auf 2° C wird das ausgeschiedene Produkt abfiltriert und mit Ethanol überwaschen.

Man erhält 39,6 g 5,10-Methylen-(6R)-tetrahydrofolsäure mit einem Gehalt von 98,6% und einem (6R)-Anteil von 99,6%; bestimmt mittels HPLC.

**Beispiel 20**

28 g von nach Beispiel 10 erhaltenem Benzolsulfonsäure-Additionssalz der (6S)-Tetrahydrofolsäure werden bei ca. 25° C unter Stickstoff in 130 ml Wasser mit Natronlauge 30% gelöst. Nach der Zugabe von 44 ml Ameisensäure 36% wird die Lösung geteilt und die eine Hälfte mit 3 g $NaBH_4$ versetzt. Nach 12 Stunden wird durch Zugabe von 10 ml Salzsäure 37% angesäuert. Das ausgeschiedene Produkt wird abfiltriert und mit Wasser und Ethanol überwaschen.

Man erhält 11 g 5-Methyl-(6S)-tetrahydrofolsäure mit einem Gehalt von 95,8% und einem (6S)-Anteil von 99,5%; bestimmt mittels HPLC.

Die andere Hälfte wird mit überschüssigem Calciumchlorid versetzt, das sich ausscheidende Produkt wird abfiltriert und mit Wasser und Ethanol überwaschen.

Man erhält 14 g 5-Formyl-(6S)-tetrahydrofolsäure-Calcium-Salz mit einem Gehalt von 96,2% und einem (6S)-Anteil von 99,7%; bestimmt mittels HPLC.

## Patentansprüche

1. Verfahren zur Herstellung von (6S)- und (6R)-Tetrahydrofolsäure sowie von deren Additionssalzen mit Sulfonsäuren oder mit Schwefelsäure, dadurch gekennzeichnet, dass man (6R,S)-Tetrahydrofolsäure mit einer Sulfonsäure oder mit Schwefelsäure umsetzt, erhaltenes Säureadditionssalz aus einem polaren Medium fraktioniert kristallisiert und, falls erwünscht, aus den erhaltenen diastereomeren Säureadditionssalzen durch Behandlung mit einer Base die (6S)-und/oder (6R)-Tetrahydrofolsäure freisetzt und isoliert.

2. Verfahren nach Anspruch 1, dadurch gekennzeichnet, dass als polares Medium vorzugsweise Wasser, oder ein Gemisch aus Wasser mit einer niedrigen, aliphatischen, wasserlöslichen Carbonsäure, insbesondere mit Essigsäure oder Milchsäure, oder ein Gemisch aus Wasser mit einem flüssigen wasserlöslichen Amid, insbesondere mit Methylpyrrolidon, Formamid, Dimethylformamid oder Dimethylacetamid verwendet wird.

3. Verfahren nach einem der Ansprüche 1 oder 2, dadurch gekennzeichnet, dass man als Sulfonsäure eine aromatische Sulfonsäure mit 6 bis 14 C-Atomen, eine araliphatische Sulfonsäure mit 7 bis 9 C-Atomen oder eine aliphatische Sulfonsäure mit 1 bis 3 C-Atomen verwendet.

4. Verfahren nach einem der Ansprüche 1, 2 oder 3, dadurch gekennzeichnet, dass man als Additionssalz das Benzolsulfonsäure-, Toluol-4-sulfonsäure- oder das Schwefelsäure-Additionssalz verwendet.

5. (6S)-Tetrahydrofolsäure-benzolsulfonat und (6R)-Tetrahydrofolsäure-benzolsulfonat.

6. (6S)-Tetrahydrofolsäure-toluol-4-sulfonat und (6R)-Tetrahydrofolsäure-toluol-4-sulfonat.

7. (6S)-Tetrahydrofolsäure-sulfat und (6R)-Tetrah-ydrofolsäuresulfat.

8. Methansulfonsäure-, Ethansulfonsäure-, Phe-nylmethansulfonsäure-, Campher-10-sulfonsäu-re-, Naphthalin-1-sulfonsäure-, Naphthalin-2-sulfonsäure-, Naphthalin-1,5-disulfonsäure-Additionssalze der (6S)-Tetrahydrofolsäure.

9. Verwendung von (6S)- oder (6R)-Tetrahydrofolsäure oder von deren Additionssalzen mit einer Sulfonsäure oder mit Schwefelsäure, hergestellt nach einem der Ansprüche 1, 2, 3 oder 4, als Ausgangsmaterial zur Herstellung von optisch reiner 5,10-Methylentetrahydrofolsäure, 5-Methyltetrahydrofolsäure oder 5-Formyltetrahydrofolsäure und deren Salzen mit starken Basen oder Säuren.

10. Verwendung von Salzen der (6S)- oder (6R)-Tetrahydrofolsäure mit Schwefelsäure oder einer Sulfonsäure hergestellt nach einem der Ansprüche 1, 2, 3 oder 4, als Bestandteil eines Arzneimittels oder als Ausgangsmaterial zur Herstellung eines Arzneimittels.

## Claims

1. Process for the preparation of (6S)- and (6R)-tetrahydrofolic acid and of their addition salts with sulphonic acids or with sulphuric acid, characterized in that (6R,S)-tetrahydrofolic acid is reacted with a sulphonic acid or with sulphuric acid, resulting acid addition salt is fractionally crystallized from a polar medium and, if desired, the (6S)- and/or (6R)-tetrahydrofolic acid is liberated from the resulting diastereomeric acid addition salts by treatment with a base and isolated.

2. Process according to Claim 1, characterized in that the polar medium used is preferably water or a mixture of water with a lower aliphatic water-soluble carboxylic acid, in particular with acetic acid or lactic acid, or a mixture of water with a liquid water-soluble amide, in particular with methylpyrrolidone, formamide, dimethylformamide or dimethylacetamide.

3. Process according to one of Claims 1 or 2, characterized in that an aromatic sulphonic acid having 6 to 14 C atoms, an araliphatic sulphonic acid having 7 to 9 C atoms or an aliphatic sulphonic acid having 1 to 3 C atoms is used as sulphonic acid.

4. Process according to one of Claims 1, 2 or 3, characterized in that the benzenesulphonic acid, toluene-4-sulphonic acid or sulphuric acid addition salt is used as addition salt.

5. (6S)-Tetrahydrofolic acid benzenesulphonate and (6R)-tetrahydrofolic acid benzenesulphonate.

6. (6S)-Tetrahydrofolic acid toluene-4-sulphonate and (6R)-tetrahydrofolic acid toluene-4-sulphonate.

7. (6S)-Tetrahydrofolic acid sulphate and (6R)-tetrahydrofolic acid sulphate.

8. Methanesulphonic acid, ethanesulphonic acid, phenylmethanesulphonic acid, camphor-10-sulphonic acid, naphthalene-1-sulphonic acid, naphthalene-2-sulphonic acid and naphthalene-1,5-disulphonic acid addition salts of (6S)-tetrahydrofolic acid.

9. Use of (6S)- or (6R)-tetrahydrofolic acid or of their addition salts with a sulphonic acid or with sulphuric acid, prepared according to one of Claims 1, 2, 3 or 4, as starting material for the preparation of optically pure 5,10-methylenetetrahydrofolic acid, 5-methyltetrahydrofolic acid or 5-formyltetrahydrofolic acid and their salts with strong bases or acids.

10. Use of salts of (6S)- or (6R)-tetrahydrofolic acid with sulphuric acid or a sulphonic acid, prepared according to one of Claims 1, 2, 3 or 4, as constituent of a medicament or as starting material for the preparation of a medicament.

**Revendications**

1. Procédé de préparation d'acide (6S)- et (6R)-tétrahydrofolique, ainsi que leurs sels d'addition avec des acides sulfoniques ou avec l'acide sulfurique, caractérisé en ce qu'on fait réagir de l'acide (6R,S)-tétrahydrofolique avec un acide sulfonique ou de l'acide sulfurique, on sépare d'un milieu polaire, par cristallisation fractionnée, le sel d'addition d'acide obtenu, et, si on le souhaite, on libère et on isole l'acide (6S)- et/ou l'acide (6R)-tétrahydrofolique, par traitement avec une base, à partir des sels d'addition d'acide diastéréoisomères obtenus.

2. Procédé selon la revendication 1, caractérisé en ce qu'on utilise comme milieu polaire de préférence l'eau, ou un mélange d'eau avec un acide carboxylique aliphatique inférieur soluble dans l'eau, en particulier avec de l'acide acétique ou de l'acide lactique, ou encore un mélange d'eau avec un amide liquide soluble dans l'eau, en particulier avec la méthylpyrrolidone, le formamide, le diméthylformamide ou le diméthylacétamide.

3. Procédé selon l'une des revendications 1 ou 2, caractérisé en ce qu'on utilise comme acide sulfonique un acide sulfonique aromatique ayant de 6 à 14 atomes de carbone, un acide sulfonique araliphatique ayant de 7 à 9 atomes de carbone ou un acide sulfonique aliphatique ayant de 1 à 3 atomes de carbone.

4. Procédé selon l'une des revendications 1, 2 ou 3, caractérisé en ce qu'on utilise comme sel d'addition le sel d'addition d'acide benzènesulfonique, d'acide toluène-4-sulfonique ou d'acide sulfurique.

5. Benzènesulfonate de l'acide (6S)-tétrahydrofolique et benzènesulfonate de l'acide (6R)-tétrahydrofolique.

6. Toluène-4-sulfonate de l'acide (6S)-tétrahydrofolique et toluène-4-sulfonate de l'acide (6R)-tétrahydrofolique.

7. Sulfate de l'acide (6S)-tétrahydrofolique et sulfate de l'acide (6R)-tétrahydrofolique.

8. Sels d'addition d'acide méthanesulfonique, d'acide éthanesulfonique, d'acide phénylméthanesulfonique, d'acide camphre-10-sulfonique, d'acide naphtalène-1-sulfonique, d'acide naphtalène-2-sulfonique, d'acide naphtalène-1,5-disulfonique, de l'acide (6S)-tétrahydrofolique.

9. Utilisation de l'acide (6S)- ou de l'acide (6R)-tétrahydrofolique ou de leurs sels d'addition d'acide sulfonique ou d'acide sulfurique, préparés selon l'une des revendications 1, 2, 3 ou 4, comme matière de départ pour préparer l'acide 5,10-méthylènetétrahydro-folique, l'acide 5-méthyltétrahydrofolique ou l'acide 5-formyltétrahydrofolique optiquement purs, ou leurs sels avec des bases fortes ou des acides forts.

10. Utilisation de sels d'acide (6S)- ou (6R)-tétrahydrofolique avec l'acide sulfurique ou avec un acide sulfonique, préparés selon l'une des revendications 1, 2, 3 ou 4, comme constituant d'un médicament ou comme matière de départ pour préparer un médicament.